# EUROPEAN PATENT SPECIFICATION

(11) **EP 3 445 229 B1**
(45) Date of publication and mention of the grant of the patent: **15.12.2021**
(21) Application number: 17786224.0
(22) Date of filing: 20.04.2017
(51) Int. Cl.: A61B 5/00, A61B 5/16, A61B 5/22, G01L 1/22, G01L 7/06, G01L 9/04, H01H 36/00, H04B 5/00, A61B 5/11

(54) **MEASUREMENT DEVICE FOR DETECTING AND MEASURING PAIN**
MESSVORRICHTUNG ZUR ERKENNUNG UND MESSUNG VON SCHMERZEN
DISPOSITIF DE MESURE DESTINÉ À LA DÉTECTION ET LA MESURE DE LA DOULEUR

(30) Priority: 22.04.2016 NO 20160684
(43) Date of publication of application: 27.02.2019
(73) Proprietor: Grasp AS, 5417 Stord (NO)
(72) Inventor: FJØSNE, Jørund, 5419 FITJAR (NO); GJØSÆTER, Tor, 5420 RUBBESTADNESET (NO); KRZYWINSKI, Aleksander, 5067 BERGEN (NO); LIE, Martin, 5054 BERGEN (NO)
(74) Representative: Håmsø Patentbyrå AS
(86) International application number: PCT/NO2017/050098
(87) International publication number: WO 2017/183994

(56) References cited:
- EP-A1- 2 359 747
- GB-A- 2 475 659
- US-A- 5 157 970
- US-A- 5 157 970
- US-A- 5 749 365
- US-A- 6 026 684
- US-A1- 2003 009 308
- US-A1- 2011 066 078
- US-A1- 2011 066 078
- US-A1- 2011 088 463
- US-A1- 2011 257 490
- US-A1- 2013 023 799
- US-A1- 2013 023 799
- US-A1- 2014 276 188

## Description

### FIELD OF THE INVENTION

The invention relates to a measurement device for detecting and measuring pain, distress or other discomfort of a user. The invention also relates to an activation system comprising such measurement device and a readout activation system.

### BACKGROUND OF THE INVENTION

Pain is one of the most frequent reasons for consulting a physician. The patient often cannot give a clearly understandable answer to the physician's question of how severe the pain is. Either the patient cannot find the right words or the physician does not understand the words used. This is inter alia because pain is a subjective sense impression, which includes sensory, cognitive and emotional aspects. An accurate measurement of pain is, however, very important, e.g. for diagnosis, when adjusting the medicinal pain therapy or in pain medication research. A plurality of methods of detecting pain have been developed to date. Most of them can be divided into two categories: One-dimensional pain scales and multidimensional pain questionnaires. The most important one-dimensional scale is the "Visual Analog Scale" (VAS). It consists of a 10cm long line whose left end is marked by "No pain" and whose right end is marked by ""Very severe pain". The patient marks the point on the line corresponding to his pain. The NRS (Numerical Rating Scale), where the selection of a number from 0-10 is communicated verbally to represent the pain, is closely related to the VAS. The VAS is used the most since it is not at all complicated and does not require any great explanations. The problem with such scales is, however, that as a person completing the scale one generally tends toward the centre and is averse to marking the extremes. On a multiple use of the VAS, users also tend toward an evaluation, which becomes closer and closer (convergence) with different pain intensity. This has the consequence that the physician acquires the impression with the detection method that only small changes are present. The most important pain questionnaire is the McGill Pain Questionnaire (MPQ). The patient is given a large choice of adjectives of which he checks the ones, which correspond to his pain. The adjectives are divided into three classes: sensory, affective and evaluative. A plurality of aspects of the pain are thus admittedly detected, but filling out takes a long time and the patient also has to understand and/or know all the adjectives to be able to complete the MPQ correctly.

There are various technical devices to detect pain, for example the apparatus disclosed in EP0,874,587B1. They are, however, mostly computerized variants of the known scales VAS or questionnaires MPQ, including their disadvantages. There are furthermore devices for applying pain stimuli for the measurement of pain thresholds and pain tolerance, e.g. the apparatus disclosed in WO2004/103230A1.

WO2009/052100A1 discloses a further apparatus for measuring pain. This apparatus has the disadvantage that the pain can only be measured with extreme imprecision and that the apparatus is only suitable to measure intestinal pain.

US2013/0046205A1 discloses a device for detecting and measuring pain felt by a person. Said device comprises a pressure or force sensor, a hollow body having an outer sleeve and an inner space, and an electronic unit for detecting the signal of the pressure or force sensor. The outer sleeve of the hollow body is embodied in such a way that it can be at least partially surrounded by a hand, the inner space of the hollow body is filled with a non-gaseous elastic material or a non-gaseous fluid, and the pressure or force sensor is arranged in such a way that the pressure of the elastic material or the fluid can be measured. When the person (the patient) feels pain he or she intuitively squeezes the hollow body thereby applying a pressure to the force sensor, which is indicated for the amount of pain the person experienced. It has been shown by experiments that these measurements are quite effective and much more accurate than the earlier described methods of registering pain. Moreover, the device may be read out by a computer in the physician's office. The logged date may then be read out, presented in a graph, and assessed by the physician, who now gets a much clearer and accurate picture of the pain history of the patient. Further technically relevant prior art documents are US 2011/066078 A1 and US 5157970 A.

Despite the latest developments in the prior art, there is still a need for further improving this technology to render the device more accurate, more robust, portable, more endurable, and more user-friendly.

### SUMMARY OF THE INVENTION

The invention has for its object to remedy or to reduce at least one of the drawbacks of the prior art, or at least provide a useful alternative to prior art.

The object is achieved through features, which are specified in the description below and in the claims that follow.

The invention is defined by the independent patent claim. The dependent claims define advantageous embodiments of the invention.

In a first aspect, the invention relates to a measurement device for detecting and measuring pain or other discomfort of a user. The measurement device comprises: i) an external housing having at least partially flexible walls and being configured for being held and squeezed by a hand of the user in response to the pain, distress, or other discomfort experienced by the user, wherein the external housing defines a first closed volume, and ii) a force sensor for detecting and measuring pressure in the first closed volume. The measurement device further comprises an inner housing, wherein the inner housing defines a second closed volume. The inner housing is suspended within the first closed volume of the external housing by means of a positioning member that fixes the inner housing to the inside of the external housing. The second closed volume comprises the force sensor, and the inner housing comprises a flexible membrane that is placed such that the flexible membrane faces the first closed volume at a first side of the flexible membrane and faces the force sensor at a second, opposite, side of the flexible membrane, wherein the flexible membrane is positioned such that there is a gap between the flexible membrane and the force sensor when no force is applied by the user to the external housing. Furthermore, at a certain predefined pressure applied by the user the flexible membrane touches the force sensor, wherein the force sensor from that instant starts to register a force.

The effects of the measurement device in accordance with the invention may be understood as follows. In the invention an internal housing is provided, wherein the internal housing comprises the force sensor. The second housing defines a second closed volume within the first closed volume. The housing further comprises a flexible membrane, which on one side faces the first closed volume, and on the other side the force sensor. The internal housing is designed such that a gap is provided between said membrane and the force sensor, when the measurement device is at rest. The advantage of this measurement device with respect to the known measurement device as discussed earlier is that the design of the invention effectively builds in a certain threshold force above which the measurement device detects and measures a pressure in the first closed volume (i.e. when the measurement device is squeezed), and below which no force is detected and measured. When a force is applied to the external housing, which increases the pressure in the first closed volume, this pressure first has to overcome a reaction force of the membrane, thereby pushing it closer to the force sensor. At a certain predefined pressure the membrane will touch the force sensor, which from that instant start to register a force. The inventor has realized that using a small threshold force will not severely deteriorate the reliability of the pain or discomfort measurement, i.e. a patient will always exceed such threshold force, in case of pain or discomfort. The inventor has also realized the user using the measurement device will typically carry it in a pocket or bag. While doing so the measurement device might be squeezed or deformed unintentionally, thereby creating the risk of unintentional registration of pain or discomfort. The invention thus conveniently reduces this risk by implementing the threshold force as described above.

A few terms used in this specification will be explained hereinafter.

Wherever the wording "closed volume" is used this is to be interpreted as a space within a housing that is closed, wherein said space may be filed with a fluid (gas or liquid), but also flexible material. When a flexible material is used, the housing obviously does not need to be fluid tight (so the word "closed" does not necessarily mean air or liquid tight), but when a liquid or gas is used it should preferably be (liquid/air) tight (or sealed). For the word "volume", other words could be used as well, such as "space", "chamber", "room", "enclosure", "region", or the like. It is submitted that all these different words have a commonality, namely that it may contain or comprise things like other components, gas, liquid, flexible material, porous material, or combinations of the above, etc.

In an embodiment of the measurement device in accordance with the invention the flexible membrane is provided with a protruding element at the second side, wherein the protruding element is configured for pressing on the force sensor when the flexible membrane is pushed towards the force sensor upon the application of the force on the external housing by the hand of the user. This embodiment conveniently increases the sensitivity of the measurement device, starting from the threshold force, because as soon as the protruding element touches the force sensor the force (pressure) applied to the force sensor is higher than what it would have been without the protruding element. Moreover, this embodiment is particularly advantageous when the flexible area is larger than the area of the force sensor.

In an embodiment of the measurement device in accordance with the invention the protruding element is designed to have a contact area when pushed to be in contact with the force sensor, wherein the contact area is designed to be a predefined factor smaller than a membrane area of the flexible membrane. The amplification factor of the force applied by the membrane is roughly determined by the area of the flexible membrane divided by the contact area of the protruding element.

In an embodiment of the measurement device in accordance with the invention the measurement device further comprises an electronic circuit coupled with the force sensor for controlling and reading out the force sensor and for storing the measurements. The electronic circuit may register when the measurement device is squeezed, how long this took place, and how much force was applied (which is an indication of the level of pain, discomfort or distress).

In an embodiment of the measurement device in accordance with the invention the measurement device further comprises a battery for supplying power to the electronic circuit. It is convenient to use a battery-powered device, when the measurement device is to be carried by a user all day long, and when it has to be used at all places he or she may be. Such battery-powered device, when being small, portable, and handheld, is also inconspicuous, which offers the user privacy, i.e. nobody will know he or she carries such device.

In an embodiment of the measurement device in accordance with the invention the electronic circuit further comprises a communication circuit for communicating registered data with a readout system. Preferably, such communication circuit comprises a wireless communication circuit, such as a Bluetooth transceiver circuit.

In an embodiment of the measurement device in accordance with the invention the electronic circuit further comprises a switching circuit, which is configured for deactivating part of the electronic circuit, preferably at least the communication circuit, during the time that the measurement device is not being read out by a readout system, and for activating the part during the time that the measurement device is to be read out by the readout system. Battery lifetime is of high importance. This problem is also acknowledged in Kanjo, E., Al-Husain, L., & Chamberlain, A. (2015). Emotions in context: examining pervasive affective sensing systems, applications, and analyses. PersUbiq Compute 19(7), (1197-1212). As pointed out in Kanjo et. al., applications used in affective sensing pose a challenge to hardware designers, as the continuous availability of the application can be a drain on available power resources. The inventors have realized that certain circuitry, such as the communication circuit (Bluetooth) can easily be switched off during normal use (non-readout) of the measurement device. The switching circuit does not necessarily need to directly switch the respective part electronically (i.e. in hardware), but could also be used to trigger the deactivation through software.

In an embodiment of the measurement device in accordance with the invention the switching circuit is controllable by a magnetic field. This embodiment conveniently cooperates with a so-called readout activation system that the inventors developed together with the measurement device. This readout activation system may be conveniently provided with one or more magnets such that the presence of a magnetic field may trigger the measurement device to activate said part of the circuitry, including the communication circuit. Similarly, the absence of the magnetic field will trigger the measurement device to deactivate said part of the circuitry.

In an embodiment of the measurement device in accordance with the invention the inner housing further comprises a space for containing respective components other than the force sensor. The electric circuit may be conveniently placed within this space, for example in the form of a printed-circuit board (PCB).

The inner housing is preferably suspended in the middle of the first closed volume such that during squeezing the user does not experience any obstructions.

In an embodiment of the measurement device in accordance with the invention the force sensor comprises a force-sensitive resistor. The force-sensitive resistor provides for a low-power solution, because it only provides for a measurement signal when a force is applied to it. Thus in rest, no power is consumed, which increases the battery lifetime significantly.

In an embodiment of the measurement device in accordance with the invention the first closed volume is at least partially filled with a fluid or an elastic material. The fluid may be a liquid or a gas. Alternatively, the first closed volume could contain an elastic porous material, which regains its form after squeezing it, wherein the pores are filled with a fluid.

In an embodiment of the measurement device in accordance with the invention the second closed volume is at least partially filled with a further fluid for converting a pressure on the flexible membrane into a pressure applied to the force sensor. In case of using a liquid in the second closed volume, special measures may be necessary for protection of the force sensor. Either the force sensor has to be able to tolerate contact with liquid or it has to be properly sealed.

In a second aspect, the invention relates to an activation system for activating reading out the measurement device. The activation system comprises the measurement device and a readout activation system configured for controlling the switching circuit for activating or deactivating the part of the electronic circuit. The activation system only relates to a group of embodiments of the measurement device of the invention, in particular those that comprise a switch for deactivating part of the electronic circuit.

In an embodiment of the activation system in accordance with claim 14 the readout activation system comprises a receiving region, such as a recess, for receiving the measurement device, and further comprises at least one magnet configured for generating a magnetic field for controlling the switching circuit for activating or deactivating the part of the electronic circuit when the measurement device is put in or taken from the receiving region, respectively. This embodiment of the activation system conveniently only relates to the embodiments of the measurement device of the invention, which comprise the switch that is activated by a magnetic field.

### BRIEF INTRODUCTION OF THE DRAWINGS

In the following is described an example of a preferred embodiment illustrated in the accompanying drawings, wherein:
- Fig. 1: shows an exploded view of an embodiment of the measurement device in accordance with the invention;
- Fig. 2: shows an enlarged cross-sectional view of part of the measurement device of Fig. 1;
- Figs. 3a-3c: illustrate some aspects of the functioning of the measurement device of the invention;
- Figs. 4a-4d: illustrate some other aspects of the functioning of the measurement device of the invention;
- Figs. 5a-5c: illustrate an embodiment of a readout activation system and a readout system for reading out said measurement device in accordance with the invention; and
- Fig. 6: illustrates the operation of the readout activation system.

### DETAILED DESCRIPTION OF THE EMBODIMENTS

The present invention relates to a measurement device for detecting and measuring pain, distress, or other discomfort of a user. The core of this measurement device is a pressure registration unit for registration of the pressure, which a user applies to the unit. The pressure registration unit will preferably be arranged to be able to register pressure in combination with time, such that it may be registered, for instance, when the user applies pressure to the unit, for how long the user applies the pressure, and how much pressure is applied. The pressure registration unit according to the invention can be used for instance in the fields of medicine, psychiatry, child welfare, and similar fields. The invention can be of a size which fits into the hand of the user. The purpose of the invention is to help the user and their therapist (or similar) to register for instance chronic pain, fear, withdrawal symptoms, and so on. Reference is made to US2013/0046205 for more background information and explanation on using such device and why it is beneficial.

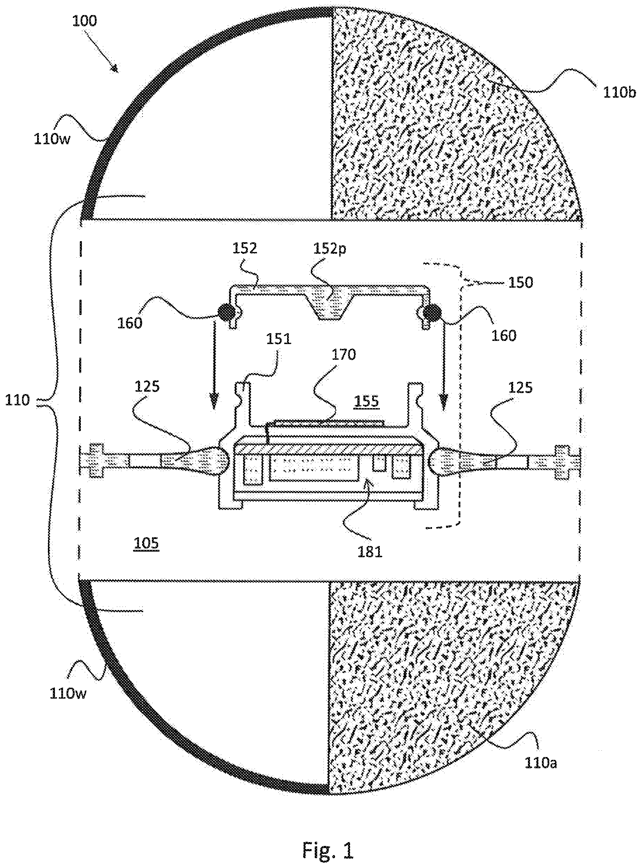

Fig. 1 shows an exploded view of an embodiment of the measurement device 100 in accordance with the invention. The measurement device 100 comprises an external housing 110 having flexible walls 110w. The external housing 110 defines (alternatively the words "encloses or isolates" could be used in this context) a first closed volume 105. In Fig. 1 it is shown that the housing 110 comprises two parts 110a, 110b that are mounted together (through gluing, screwing, clamping, or the like). The measurement device 100 further comprises an internal housing 150, which is suspended within the external housing 110 by means of a positioning member 125 as shown. As the external housing is flexible, the positioning member 125 is preferably also flexible. When the respective parts 110a, 110b of the external housing are mounted together, they effectively hold the positioning member 125 in place. The internal housing 150 comprises a first part 151, which may be non-flexible or at least much less flexible than the external housing 110. The first part 151 of the internal housing partially defines a first space 155, which comprises a force sensor 170, which preferably is a force-sensitive resistor (FSR), because of its low power consumption advantage. The first space 155 is further sealed off by a flexible membrane 152, which in this embodiment is to be clicked over said first part 151 of the internal housing 150 and held in place with an O-ring 160, as illustrated. The membrane 152 in this embodiment is provided with a protruding element 152p, which is advantageous as discussed in the introduction of this specification.

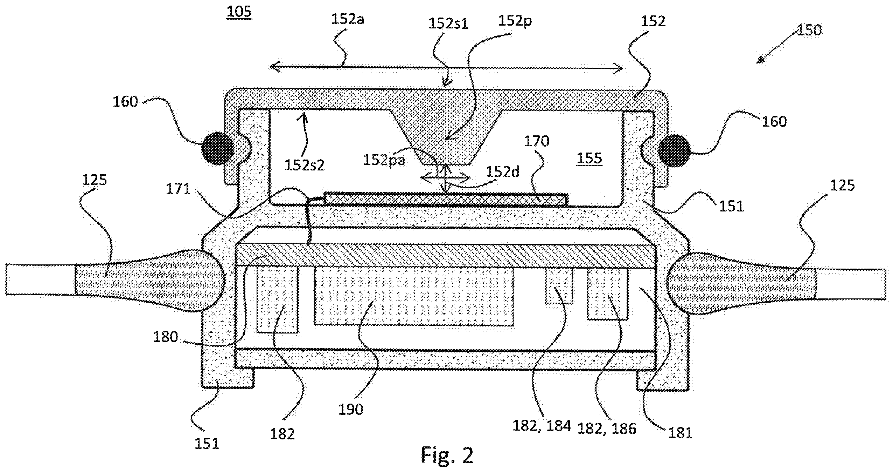

Fig. 2 shows an enlarged cross-sectional view of the part of the measurement device of Fig. 1. In the figure, the flexible membrane 152 has been mounted on the first part 151 of the internal housing 150. By mounting said flexible membrane 152, the second closed volume 155 (earlier mentioned first space) is formed. It is illustrated how the flexible membrane 152 faces the first closed volume 105 on a first side 152s1 thereof, and the force sensor 170 on a second, opposite, side 152s2 thereof. The figure further illustrates that there is a gap 152d between said protruding part 152p and the force sensor 170. Another aspect that Fig. 2 illustrates is the amplification of the pressure/force applied to the membrane 152. The pressure in the first closed volume 105 presses on an area 152a with the size as illustrated by the arrow. Under this pressure the membrane 152 may bend towards the force sensor 170. As soon as the protruding element 152p of the membrane touches the force sensor 170 it is the contact area 152pa of the protruding element 152p, as illustrated by the other arrow, which will transfer the force to the force sensor 170. This force/pressure is effectively an amplified version of the force applied to the membrane 152. The amplification factor is typically in the order of the ratio between the membrane area 152a and the contact area 152pa of the protruding element 152.

Fig. 2 further illustrates that the force sensor 170 is connected to a printed-circuit board (PCB) 180 via a connecting wire (or wires) 171. The PCB 180 is provided in a second space 181 of the internal housing 150 as illustrated. The PCB 180 is provided with en electronic circuit 182 (i.e. an integrated circuit), which may comprise a processor or control unit for controlling and reading out the force sensor 170 and storage circuitry for storing said measurements). The electronic circuit 182 in this embodiment also comprises a switching circuit 184 and a communication circuit 186. The communication circuit 186 may be a Bluetooth transceiver circuit, which as such is well-known to the person skilled in the art. The electronic circuit is power by a battery 190. The switching circuit 184 has been added to facilitate deactivating part of the electronic circuit 182, in particular the communication circuit 186, for reasons as earlier discussed, i.e. save power and thereby increase battery lifetime.

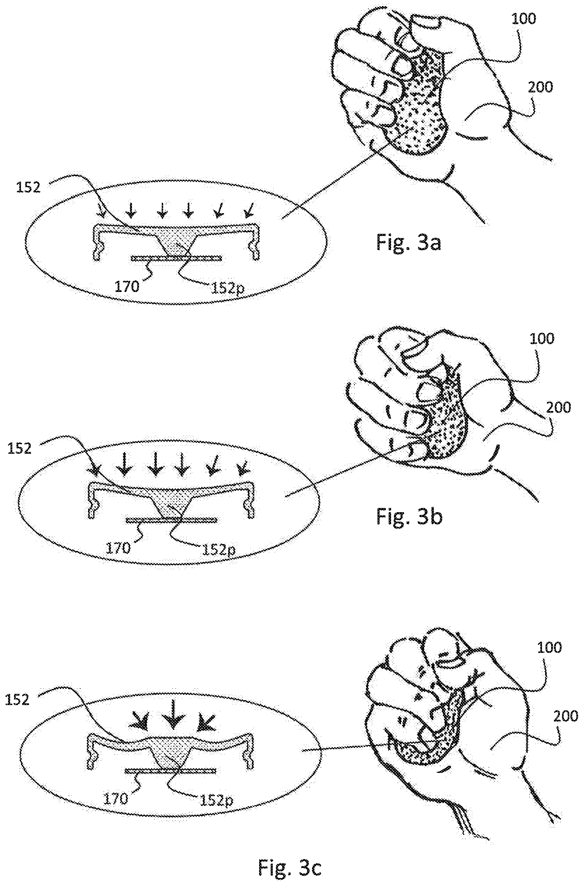

Figs. 3a-3c illustrate some aspects of the functioning of the measurement device of the invention. In Fig. 3a the hand 200 of the user applies the so-called threshold pressure to the measurement device 100. In this situation the flexible membrane 152, or the protruding element 152p if present, barely touches the force sensor 170, i.e. it begins to apply a small force when the force applied to the measurement device 100 is further increased. Fig. 3b shows the situation where the user applies a medium force to the measurement device. In this situation the flexible membrane 152, or the protruding element 152p if present, applies a force to the force sensor 170. Fig. 3c shows the situation where the user applies a maximum force to the measurement device. With "maximum force" it is meant the maximum force that can be registered, not the maximum force that could be applied. The force sensor 170 in this situation outputs its maximum signal level to the electronic circuit 180.

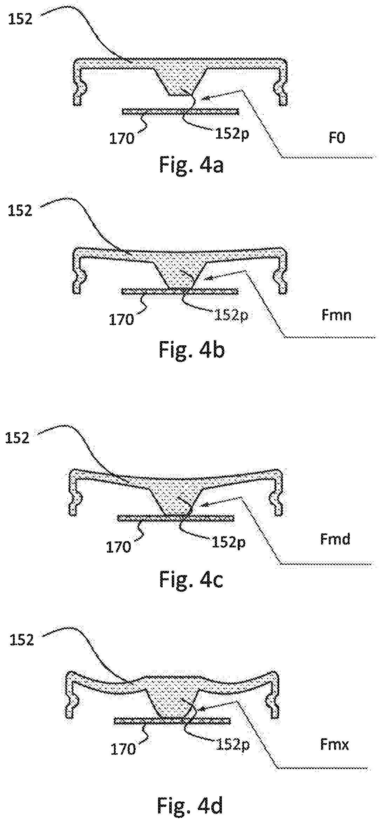

Figs. 4a-4d illustrate some other aspects of the functioning of the measurement device of the invention. Fig. 4a shows the equilibrium situation, where the user does not apply a pressure to the measurement device 100. Consequently, the membrane 152 applies a zero force F0 to the force sensor 170 (although there is still the pressure in the 155 second closed volume, which applies a pressure on the force sensor 170). Fig. 4b shows the situation which complies with Fig. 3a. A minimum force Fmn is applied to the force sensor 170. Fig. 4c shows the situation that complies with Fig. 3b. A medium force Fmd is applied to the force sensor 170. Fig. 4d shows the situation that complies with Fig. 3c. The maximum registerable force Fmx is applied to the force sensor 170. The reason for differentiating between F0 and Fmn is that the force sensor may be very sensitive (for instance when an FSR is used). Thus, in case of small pressure variations in the situation of Fig. 4a having the gap between the membrane 152 and the force sensor 170 nothing will be measured and registered by the measurement device 100. In the situation of Fig. 4b however the pressure is registered by the measurement device 100. Expressed differently, the small-signal behaviour in these two situations is very different.

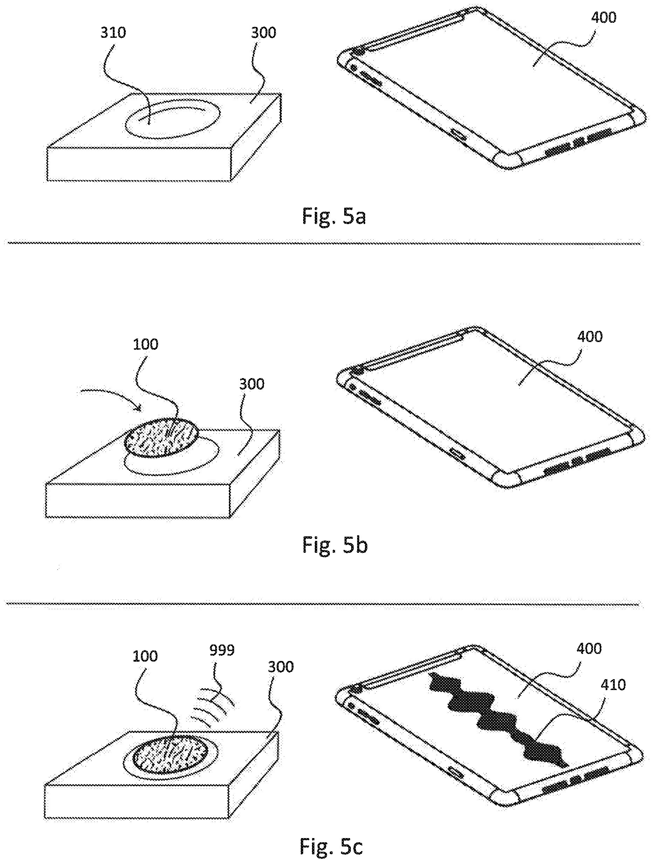

Figs. 5a-5c illustrate an embodiment of a readout activation system 300 and a readout system for reading out said measurement device in accordance with the invention. The focus of the inventor has been to increase battery lifetime as much as possible. The communication circuit 182 only needs to be active when the measurement device 100 is read out by a readout system (i.e. laptop, table, PC, smartphone) 400. As discussed earlier the measurement device 100 may comprise a switching circuit for (triggering) deactivating said communication 182 (and/or other parts). In a preferred embodiment the deactivation is done in software. The measurement device 100 is configured such, that when it is put in the readout activation device 300 (also called the crib) it will broadcast and be ready for connection for a predefined time, for instance 30 seconds. After this time has lapsed, the measurement device 100 will return to its sleep mode again (arranged in software) even if the measurement device 100 remains in the crib 300.

In Fig. 5a there is provided the readout activation system 300, which comprises in this embodiment a receiving region 310 (such as a recess) for receiving the measurement device 100 in accordance with the invention. When for example Bluetooth technology is used for wireless communication, the readout activation system 300 is preferably put within Bluetooth communication reach of the readout system 400. In Fig. 5b the measurement device 100 is put into the recess 310. In Fig. 5c the measurement device 100 is activated by the readout activation system 300 and transmits its registered data to the readout system 400 via a wireless communication signal 999 as schematically illustrated. The registered data may be shown on the readout system 400 in the form of a graph 410 for example. The physician may subsequently interpret the registered data, which forms an indication of the pain, distress or other discomfort of the user over a certain period.

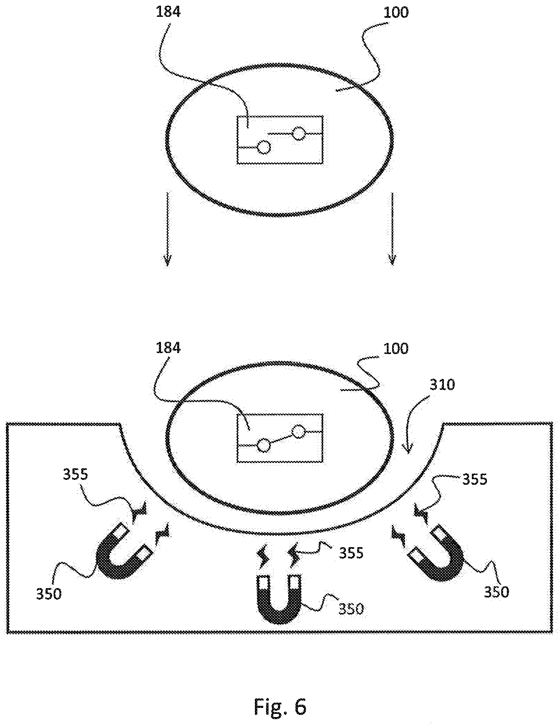

Fig. 6 illustrates the operation of the readout activation system 300. This figure is very schematic in order to facilitate understanding of the principle. Near the recess 310 of the readout activation device 300 there is provided a plurality (but at least one) of magnets 350 as illustrated. These could be permanent magnets, but also other magnets are possible. These magnets 350 generate a magnetic field 355, which subsequently trigger the earlier-discussed switching circuit 184 to activate the communication circuit (not shown in Fig. 6), for instance by closing or opening when the measurement device 100 is placed in the recess 310. The switching circuit 184 be used to directly switch (on or off) the respective part electronically (i.e. in hardware), but it could also be used to trigger the deactivation through software.

It must be stressed that only one embodiment of the invention has been illustrated in the figures. However, many variations of the measurement device in accordance with the invention are possible without deviating from the scope of protection as defined by the claims. It must be stressed that the protruding element of the membrane is optional, yet results in an advantage when used. In addition, the measurement device may have other forms than illustrated. Furthermore, the second closed volume may be provided outside the first closed volume if so desired, wherein the flexible membrane then effectively forms the interface between both volumes.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The mere fact that certain measures are recited in mutually different dependent claims does not indicate that a combination of these measures cannot be used to advantage. In the device claim enumerating several means, several of these means may be embodied by one and the same item of hardware.

## Claims

1. Measurement device (100) for detecting and measuring pain, distress, or other discomfort of a user, the measurement device (100) comprising:
- an external housing (110) having at least partially flexible walls (110w) and being configured for being held and squeezed by a hand (200) of the user in response to the pain, distress, or other discomfort experienced by the user, wherein the external housing (110) defines a first closed volume (105), and
- a force sensor (170) for detecting and measuring pressure in the first closed volume (105), **characterised in that** the measurement device (100) further comprises an inner housing (150), wherein the inner housing (150) defines a second closed volume (155), wherein the inner housing (150) is suspended within the first closed volume (105) of the external housing (110) by means of a positioning member (125) that fixes the inner housing (150) to the inside of the external housing (110), the second closed volume (155) comprising the force sensor (170), wherein the inner housing (150) comprises a flexible membrane (152) that is placed such that the flexible membrane (152) faces the first closed volume (105) at a first side (152s1) of the flexible membrane (152) and faces the force sensor (170) at a second, opposite, side (152s2) of the flexible membrane (152), wherein the flexible membrane (152) is positioned such that there is a gap (152d) between the flexible membrane (152) and the force sensor (170) when no force is applied by the user to the external housing (110), and that at a certain predefined pressure applied by the user the flexible membrane (152) touches the force sensor (170), wherein the force sensor (170) from that instant starts to register a force.

2. The measurement device (100) according to claim 1, wherein the flexible membrane (152) is provided with a protruding element (152p) at the second side (152s2), wherein the protruding element (152p) is configured for pressing on the force sensor (170) when the flexible membrane (152) is pushed towards the force sensor (170) upon the application of the force on the external housing (110) by the hand (200) of the user.

3. The measurement device (100) according to claim 2, wherein the protruding element (152p) is designed to have a contact area (152pa) when pushed to be in contact with the force sensor (170), wherein the contact area (152pa) is designed to be a predefined factor smaller than a membrane area (152a) of the flexible membrane (152).

4. The measurement device (100) according to any one of the preceding claims, wherein the measurement device (100) further comprises an electronic circuit (182) coupled with the force sensor (170) for controlling and reading out the force sensor (170) and for storing the measurements.

5. The measurement device (100) according to claim 4, wherein the measurement device (100) further comprises a battery (190) for supplying power to the electronic circuit (182).

6. The measurement device (100) according to any one of the preceding claims, wherein the electronic circuit (182) further comprises a communication circuit (186) for communicating registered data with a readout system (400).

7. The measurement device (100) according to claim 6, wherein the electronic circuit (182) further comprises a switching circuit (184), which is configured for deactivating part (186) of the electronic circuit (182), preferably at least the communication circuit (186), during the time that the measurement device (100) is not being read out by a readout system (400), and for activating the part (186) during the time that the measurement device (100) is to be read out by the readout system (400).

8. The measurement device (100) according to claim 7, wherein the switching circuit (184) is controllable by a magnetic field (355).

9. The measurement device (100) according to any one of the preceding claims, wherein the inner housing (150) further comprises a space (181) for containing respective components other than the force sensor (170).

10. The measurement device (100) according to any one of the preceding claims, wherein the force sensor (170) comprises a force-sensitive resistor.

11. The measurement device (100) according to any one of the preceding claims, wherein the first closed volume (105) is at least partially filled with a fluid or an elastic material.

12. The measurement device (100) according to claim 1, wherein the second closed volume (155) is at least partially filled with a further fluid for converting a pressure on the flexible membrane (152) into a pressure applied (F0, Fmn, Fmd, Fmx) to the force sensor (170).

13. Activation system for activating reading out the measurement device (100), the activation system comprising:
- the measurement device (100) in accordance with claim 7 or any one of claims 8 to 12 in as far as directly or indirectly dependent on claim 7, and
- a readout activation system (300) configured for controlling the switching circuit (184) for activating or deactivating the part (186) of the electronic circuit (182).

14. The activation system according to claim 13 with the measurement device (100) in accordance with claim 8 or any one of claims 9 to 12 in as far as directly or indirectly dependent on claim 8, wherein the readout activation system (300) comprises a receiving region (310), such as a recess, for receiving the measurement device (100), and further comprising at least one magnet (350) configured for generating a magnetic field (355) for controlling the switching circuit (184) for activating or deactivating the part (186) of the electronic circuit (182) when the measurement device (100) is put in or taken from the receiving region (310), respectively.

## Patentansprüche

1. Messvorrichtung (100) zur Ermittlung und Messung von Schmerz, Not oder anderem Unwohlsein eines Benutzers, wobei die Messvorrichtung (100) aufweist:
- ein Aussengehäuse (110), aufweisend mindestens teilweise flexible Wände (110w) und ausgebildet, um von einer Hand (200) des Benutzers gehalten und zusammengepresst zu werden als Reaktion auf den Schmerz, die Not oder anderes vom Benutzer erlebtes Unwohlsein, wobei das Aussengehäuse (110) ein erstes geschlossenes Volumen (105) definiert, und
- einen Kraftsensor (170) zur Ermittlung und Messung von Druck im ersten geschlossenen Volumen (105), **dadurch gekennzeichnet, dass** die Messvorrichtung (100) ferner ein Innengehäuse (150) aufweist, wobei das Innengehäuse (150) ein zweites geschlossenes Volumen (155) definiert, wobei das Innengehäuse (150) innerhalb des ersten geschlossenen Volumens (105) des Aussengehäuses (110) mittels eines Positionierungselements (125), welches das Innengehäuse (150) an der Innenseite des Aussengehäuses (110) befestigt, aufgehängt ist, wobei das zweite geschlossene Volumen (155) den Kraftsensor (170) aufweist, wobei das Innengehäuse (150) eine flexible Membran (152) aufweist, welche derart platziert ist, dass die flexible Membran (152) an einer ersten Seite (152s1) der flexiblen Membran dem ersten geschlossenen Volumen (105) zugewandt ist, und an einer zweiten, gegenüberliegenden Seite (152s2) der flexiblen Membran (152) dem Kraftsensor (170) zugewandt ist, wobei die flexible Membran (152) derart positioniert ist, dass sich ein Zwischenraum (152d) zwischen der flexiblen Membran (152) und dem Kraftsensor (170) ergibt, wenn keine Kraft vom Benutzer auf das Aussengehäuse (110) einwirkt, und dass bei einem bestimmten vordefinierten, vom Benutzer angewandten Druck die flexible Membran (152) den Kraftsensor (170) berührt, wobei der Kraftsensor (170) von dem Moment an beginnt, eine Kraft zu verzeichnen.

2. Messvorrichtung (100) gemäss Anspruch 1, wobei die flexible Membran (152) mit einem vorstehenden Element (152p) an der zweiten Seite (152s2) ausgebildet ist, wobei das vorstehende Element (152p) ausgebildet ist, auf den Kraftsensor (170) zu drücken, wenn die flexible Membran (152) zum Kraftsensor (170) hin gedrückt wird als Folge der Einwirkung von Kraft auf das Aussengehäuse (110) durch die Hand (200) des Benutzers.

3. Messvorrichtung (100) gemäss Anspruch 2, wobei das vorstehende Element (152p) ausgebildet ist, eine Kontaktfläche (152pa) aufzuweisen, wenn es in einen Kontakt mit dem Kraftsensor (170) gedrückt wird, wobei die Kontaktfläche (152pa) ausgebildet ist, um einen vordefinierten Faktor kleiner zu sein als eine Membranfläche (152a) der flexiblen Membran (152).

4. Messvorrichtung (100) gemäss einem der vorhergehenden Ansprüche, wobei die Messvorrichtung (100) ferner einen elektronischen Schaltkreis (182) aufweist, welcher mit dem Kraftsensor (170) verbunden ist, zur Steuerung und zum Ablesen des Kraftsensors (170) und zur Speicherung der Messungen.

5. Messvorrichtung (100) gemäss Anspruch 4, wobei die Messvorrichtung (100) ferner eine Batterie (190) aufweist, zur Versorgung des elektronischen Schaltkreises (182) mit Strom.

6. Messvorrichtung (100) gemäss einem der vorhergehenden Ansprüche, wobei der elektronische Schaltkreis (182) ferner einen Kommunikationsschaltkreis (186) aufweist, zum Austausch von ermittelten Daten mit einem Ablesesystem (400).

7. Messvorrichtung (100) gemäss Anspruch 6, wobei der elektronische Schaltkreis (182) ferner einen Schalterstromkreis (184) aufweist, welcher ausgebildet ist, um einen Teil (186) des elektronischen Schaltkreises (182), vorzugsweise mindestens den Kommunikationsschaltkreis (186) zu deaktivieren, während der Zeit, in welcher die Messvorrichtung (100) nicht von einem Ablesesystem (400) abgelesen wird, und zur Aktivierung des Teils (186) während der Zeit, in welcher die Messvorrichtung (100) vom Ablesesystem (400) abgelesen wird.

8. Messvorrichtung (100) gemäss Anspruch 7, wobei der Schalterstromkreis (184) durch ein Magnetfeld (355) steuerbar ist.

9. Messvorrichtung (100) gemäss einem der vorhergehenden Ansprüche, wobei das Innengehäuse (150) ferner einen Raum (181) zur Aufnahme von jeweiligen Komponenten ausser des Kraftsensors (170) aufweist.

10. Messvorrichtung (100) gemäss einem der vorhergehenden Ansprüche, wobei der Kraftsensor (170) einen kraftsensitiven Widerstand aufweist.

11. Messvorrichtung (100) gemäss einem der vorhergehenden Ansprüche, wobei das erste geschlossene Volumen (105) mit einem Fluid oder einem elastischen Material mindestens teilweise gefüllt ist.

12. Messvorrichtung (100) gemäss Anspruch 1, wobei das zweite geschlossene Volumen (155) mit einem weiteren Fluid mindestens teilweise gefüllt ist, zur Umwandlung eines Druckes auf der flexiblen Membran (152) in einen auf den Kraftsensor (170) einwirkenden Druck (F0, Fmn, Fmd, Fmx).

13. Aktivierungssystem zur Aktivierung des Ablesens der Messvorrichtung (100), wobei das Aktivierungssystem aufweist:
- die Messvorrichtung (100) gemäss Anspruch 7 oder einem der Ansprüche 8 bis 12, sofern direkt oder indirekt abhängig von Anspruch 7, und
- ein Ablese-Aktivierungssystem (300), welches ausgebildet ist, um den Schalterstromkreis (184) zu steuern, zur Aktivierung oder Deaktivierung des Teils (186) des elektronischen Schaltkreises (182).

14. Aktivierungssystem gemäss Anspruch 13 mit der Messvorrichtung (100) gemäss Anspruch 8 oder einem der Ansprüche 9 bis 12, sofern direkt oder indirekt abhängig von Anspruch 8, wobei das Ablese-Aktivierungssystem (300) einen Aufnahmebereich (310) aufweist, wie beispielsweise eine Ausnehmung, zur Aufnahme der Messvorrichtung (100) und ferner aufweisend mindestens einen Magnet (350), welcher ausgebildet ist, um ein Magnetfeld (355) zu erzeugen, zur Steuerung des Schalterstromkreises (184) zur Aktivierung oder Deaktivierung des Teils (186) des elektronischen Schaltkreises (182), wenn die Messvorrichtung (100) in den Aufnahmebereich (310) hinein gesetzt bzw. daraus heraus genommen wird.

## Revendications

1. Dispositif de mesure (100) pour détecter et mesurer la douleur, la détresse ou tout autre inconfort d'un utilisateur, le dispositif de mesure (100) comprenant :
- un boîtier extérieur (110) ayant des parois au moins partiellement flexibles (110w) et étant configuré pour être tenu et pressé par une main (200) de l'utilisateur, en réponse à la douleur, la détresse ou tout autre inconfort perçu par l'utilisateur, dans lequel le boîtier extérieur (110) définit un premier volume fermé (105), et
- un capteur de force (170) pour détecter et mesurer la pression dans le premier volume fermé (105), **caractérisé en ce que** le dispositif de mesure (100) comprend en outre un boîtier intérieur (150), dans lequel le boîtier intérieur (150) définit un deuxième volume fermé (155), dans lequel le boîtier intérieur (150) est suspendu à l'intérieur du premier volume fermé (105) du boîtier extérieur (110) au moyen d'un organe de positionnement (125) qui fixe le boîtier intérieur (150) à l'intérieur du boîtier extérieur (110), le deuxième volume fermé (155) comprenant le capteur de force (170), dans lequel le boîtier intérieur (150) comprend une membrane flexible (152) qui est placée de telle sorte que la membrane flexible (152) fait face au premier volume fermé (105) au niveau d'un premier côté (152s1) de la membrane flexible (152) et fait face au capteur de force (170) au niveau d'un deuxième côté opposé (152s2) de la membrane flexible (152), dans lequel la membrane flexible (152) est positionnée de telle sorte qu'il existe un espace (152d) entre la membrane flexible (152) et le capteurde force (170) lorsqu'aucune force n'est appliquée par l'utilisateur sur le boîtier extérieur (110) et qu'à une certaine pression prédéfinie appliquée par l'utilisateur, la membrane flexible (152) touche le capteur de force (170), dans lequel à partir de cet instant, le capteur de force (170) commence à enregistrer une force.

2. Le dispositif de mesure (100) selon la revendication 1, dans lequel la membrane flexible (52) est munie d'un élément en saillie (152p) au niveau du deuxième côté (152s2), dans lequel l'élément en saillie (152p) est configuré pour presser sur le capteur de force (170) lorsque la membrane flexible (152) est poussée vers le capteur de force (170) lors de l'application de la force sur le boîtier extérieur (110) par la main (200) de l'utilisateur.

3. Le dispositif de mesure (100) selon la revendication 2, dans lequel l'élément en saillie (152p) est conçu pour avoir une zone de contact (152pa) lorsqu'il est poussé pour être en contact avec le capteur de force (170), dans lequel la zone de contact (152pa) est conçue pour être plus petite d'un facteur prédéfini à une surface de membrane (152a) de la membrane flexible (152).

4. Le dispositif de mesure (100) selon l'une quelconque des revendications précédentes, dans lequel le dispositif de mesure (100) comprend en outre un circuit électronique (182) couplé au capteur de force (170) pour commander et lire le capteur de force (170) et pour stocker les mesures.

5. Le dispositif de mesure (100) selon la revendication 4, dans lequel le dispositif de mesure (100) comprend en outre une pile (190) pour alimenter le circuit électronique (182).

6. Le dispositif de mesure (100) selon l'une quelconque des revendications précédentes, dans lequel le circuit électronique (182) comprend en outre un circuit de communication (186) pour communiquer des données enregistrées avec un système de lecture (400).

7. Le dispositif de mesure (100) selon la revendication 6, dans lequel le circuit électronique (182) comprend en outre un circuit de commutation (184), qui est configuré pour désactiver une partie (186) du circuit électronique (182), de préférence au moins le circuit de communication (186), durant le temps où le dispositif de mesure (100) n'est pas lu par un système de lecture (400), et pour activer la partie (186) durant le temps où le dispositif de mesure (100) doit être lu par le système de lecture (400).

8. Le dispositif de mesure (100) selon la revendication 7, dans lequel le circuit de commutation (184) est contrôlable par un champ magnétique (355).

9. Le dispositif de mesure (100) selon l'une quelconque des revendications précédentes, dans lequel le boîtier intérieur (150) comprend en outre un espace (181) pour contenir des composants respectifs autres que le capteur de force (170).

10. Le dispositif de mesure (100) selon l'une quelconque des revendications précédentes, dans lequel le capteur de force (170) comprend un condensateur sensible à la force.

11. Le dispositif de mesure (100) selon l'une quelconque des revendications précédentes, dans lequel le premier volume fermé (105) est au moins partiellement rempli d'un fluide ou d'un matériau élastique.

12. Le dispositif de mesure (100) selon la revendication 1, dans lequel le deuxième volume fermé (155) est au moins partiellement rempli d'un autre fluide pour convertir une pression sur la membrane flexible (152) en une pression appliquée (F0, Fmn, Fmd, Fmx) au capteur de force (170).

13. Système d'activation pour activer une lecture du dispositif de mesure (100), le système d'activation comprenant :
- le dispositif de mesure (100) selon la revendication 7 ou une quelconque des revendications 8 à 12 dans la mesure où elle dépend directement ou indirectement de la revendication 7, et
- un système d'activation de lecture (300) configuré pour commander le circuit de commutation (184) pour activer ou désactiver la partie (186) du circuit électronique (182).

14. Le système d'activation selon la revendication 13 avec le dispositif de mesure (100) selon la revendication 8 ou une quelconque des revendications 9 à 12 dans la mesure où elle dépend directement ou indirectement de la revendication 8, dans lequel le système d'activation de lecture (300) comprend un domaine de réception (310), tel qu'un évidement, pour recevoir le dispositif de mesure (100), et comprenant en outre au moins un aimant (350) configuré pour générer un champ magnétique (355) pour commander le circuit de commutation (184) pour activer ou désactiver la partie (186) du circuit électronique (182) lorsque le dispositif de mesure (100) est introduit dans, ou prélevé du, domaine de réception (310), respectivement.
